# EUROPEAN PATENT APPLICATION

(11) **EP 4 631 549 A1**
(43) Date of publication of application: **15.10.2025**
(21) Application number: 24315124.8
(22) Date of filing: 08.04.2024
(51) Int. Cl.: A61M 5/142, A61M 5/145

(54) **DRUG DELIVERY DEVICE AND METHOD FOR CONTROLLING THIS DRUG DELIVERY DEVICE**

(71) Applicant: Becton Dickinson France, 38800 Le Pont-de-Claix (FR)
(72) Inventor: Delobelle, Vincent, 38420 DOMENE (FR); Besson, Nicolas, 38650 TREFFORT (FR); Bernède, Gilles, 74930 ARBUSIGNY (FR); Bouyahiaoui, Salim, 74000 ANNECY (FR)
(74) Representative: Germain Maureau

(57) **Abstract**

The drug delivery device (1) includes: a housing (11), a reservoir (15) configured for storing a medical product, an injection mechanism (2) configured for allowing injection of the medical product into an injection site, a pump unit (3) for pumping the medical product from the reservoir (15) and expelling this medical product towards the injection site, a motor unit (4) for driving the pump unit (3), and a movement converter (5) including a driving shaft (51) and coupled to the motor unit (4) and a coupling shaft (58) coupled to a piston (310) of the pump unit (3). The movement converter (5) is configured for converting a one-way rotation of the driving shaft (51) into a two-way rotation of the coupling shaft (58).

## Description

The present invention relates to a drug delivery device and to a method for controlling this drug delivery device.

In this application, the distal end of a component or of a device is to be understood as meaning the end furthest from a motor of the drug delivery device, with respect to an axis X parallel to the motor shaft, and the proximal end is to be understood as meaning the end closest from the motor, with respect to the axis X. Likewise, in this application, the "distal direction" is to be understood as meaning the direction away from the motor along the axis X, and the "proximal direction" is to be understood as meaning the opposite direction towards along the axis X.

Drug delivery devices typically enable to deliver a medical product stored in a reservoir to an injection site. Some drug delivery devices, such as wearable injectors, include a pump for pumping the medical product from the reservoir and move the medical product towards the injection site. Wearable injectors are injectors configured to be worn by a patient, and more specifically attached to the patient's skin, during a predetermined time period, usually ranging from several minutes or hours to several days. The wearable injectors are configured to deliver a predetermined volume of medical product at a predetermined flow rate and at a predetermined time after activation.

The medical product is prefilled in a reservoir of the wearable injection system or is transferred from a prefilled syringe or a vial to a reservoir of the wearable injection by a healthcare worker, such as a nurse, before activation and application of the wearable injector onto the patient's skin. The prefilled syringe is typically contained in a kit packaging which also contains the wearable injector.

Wearable injectors typically include a reservoir, such as a cartridge, a syringe or a bag, configured for containing the medical product, an injection mechanism including for instance an injection needle or a catheter for fluidly connecting the reservoir to an injection site, and a pump activated by a motor for expelling the medical product contained within the reservoir into an injection site, via the injection mechanism, at the predetermined time, volume and flow rate.

The pump typically includes a piston which has a back-and-forth axial movement, corresponding to the intake phase and discharge phase of the pump cycle. The piston is driven by a motor shaft. The motor shaft has to rotate in one direction to move the piston in a first axial direction, then the motor shaft has to stop and change direction, i.e. to turn in the opposite way, in order to cause the piston to move in a second opposite axial direction. In other words, in order to accomplish a full pump cycle, the motor shaft has to i) turn in a first direction, ii) stop, iii) turn in a second opposte direction, iv) stop and begin a new cycle.

However, this alternated rotation of the motor shaft requires to repeatedly increase and then pace down the rotation speed of the motor shaft. This needs an accurate, and expensive, control of the motor shaft. Besides, alternating the rotational direction of the motor shaft involves that the gearbox of the motor starts and stops at the same position in each cycle. This may lead to premature aging and failure of the gears, causing dysfunction of the drug delivery device. The premature aging of the motor or the gearbox is all the more problematic when the drug delivery device is reusable.

There is therefore a need for a drug delivery device that alleviates some or all of the aforementioned drawbacks. Specifically, there is a need for a drug delivery device that prevents premature aging and failure of its motor unit. There is also a need for a drug delivery device which does not require accurate control of the motof shaft.

In this context, an aspect of the invention is a drug delivery device including:
a housing
a reservoir configured for storing a medical product
an injection mechanism configured for allowing injection of the medical product into an injection site
a pump unit for pumping the medical product from the reservoir and expelling this medical product towards the injection site
a motor unit for driving the pump unit,
a movement converter including a driving shaft coupled to the motor unit and a coupling shaft coupled to a piston of the pump unit, wherein the movement is converter configured for converting a one-way rotation of the driving shaft into a two-way rotation of the coupling shaft.

Thus, the driving shaft, which may be the motor shaft or which may be coupled to the motor shaft, can continuously rotate in a single way. The drug delivery device of the invention does not need a costly control of the driving shaft position. Besides, the drug delivery device of the invention has a better lifetime since the driving shaft does not have to reciprocate in one rotational direction and the other.

The pump unit includes a semi rotary back-and-forth pump.

The device of the invention may further include some or all of the features listed below.

The piston of the pump unit may be configured to rotate according to a predetermined angle equal to or greater than 180°, preferably comprised between [190°-210°].

That is, the piston rotates according to this predetermined angle in one way, and then rotates according to this predetermined angle in the opposite way to execute a full pump cycle. The movement converter of the invention enables to convert the one-way continuous rotation of the motor unit into an alternated two-way rotation of the piston of the pump unit limited to this predetermined angle.

In an embodiment, the movement converter includes an upstream crank fixed to the driving shaft, a downstream crank fixed to the coupling shaft, and one or more connecting rods rotatably connecting the upstream crank to the downstream crank.

The one or more connecting rods may include three successive connecting rods.

In an embodiment, the one or more connecting rods include a first connecting rod rotatably mounted on the upstream crank around an axis X2, a second connecting rod rotatably mounted on the first connecting rod an axis X3, and a third connecting rod rotatably mounted on the second connecting rod around an axis X5 and rotatably mounted on the downstream crank around an axis X6.

Possibly, the second connecting rod is rotatably mounted on the housing around an axis X4.

That is, the second connecting rod is rotatably mounted around a fixed rotation axis X4. The housing may be the pump housing, the motor housing or any other part fixedly connected to the housing of the drug delivery device.

The axis X1, X2, X3, and X5, X6 are movable with respect to the housing, whereas the axis X4 is fixed. The axis X1 of the driving shaft and the axis X7 of the coupling shaft may also be fixed with respect to the housing.

In an embodiment, the second connecting rod includes a first portion extending between the axis X4 and the axis X3, and a second portion, inclined with respect to the first portion, the second portion extending between the axis X3 and the axis X5.

In an embodiment, the first connecting rod is disposed between the upstream crank and the second connecting rod.

In an embodiment, the third connecting rod is disposed between the second connecting rod and the downstream crank.

In an embodiment, the driving shaft and the coupling shaft are parallel.

In an embodiment, the driving shaft and the coupling shaft are coaxial.

Preferably, the second connecting rod is disposed between the first connecting rod and the second connecting rod.

In an embodiment, the driving shaft and the coupling shaft are offset.

Preferably, the first connecting rod and the third connecting rod are disposed at a same side of the second connecting rod.

Another aspect of the invention is a method for method for setting the above-described drug delivery device, the method including the steps of:
determining a targeted flow rate for the medical product at a catheter of the drug delivery device,
setting a rotation speed profile for the driving shaft.

The invention and the advantages arising therefrom will clearly emerge from the detailed description that is given below with reference to the appended drawings as follows :
- Figure 1A is a perspective view of a drug delivery device according to an embodiment of the invention, illustrated with a see-through housing for clarity,
- Figure 1B is an exploded view of the drug delivery device of Figure 1A,
- Figure 2A is a perspective view of a pump unit of a drug delivery device according to an embodiment of the invention,
- Figure 2B is an exploded view of the pump unit of Figure 2A,
- Figures 3A to 3D are perspective views illustrating the operation of a pump unit of a drug delivery device according to an embodiment of the invention, illustrated with a see-through pump housing,
- Figure 4 is a schematic view of the inside of a drug delivery device according to one embodiment of the invention,
- Figure 5 is a perspective view illustrating the pump unit, the converter and the motor unit of the drug delivery device of Figure 4,
- Figure 6 is a top view illustrating the pump unit, the converter and the motor unit of the drug delivery device of Figure 4,
- Figure 7 is a front view of the converter of the drug delivery device of Figure 4,
- Figures 8A to 8D illustrate successive steps of operation of the converter of the drug delivery device of Figure 4,
- Figure 9 is a schematic view of the inside of a drug delivery device according to another embodiment of the invention,
- Figure 10 is a perspective view illustrating the pump unit, the converter and the motor unit of the drug delivery device of Figure 9,
- Figure 11 is a top view illustrating the pump unit, the converter and the motor unit of the drug delivery device of Figure 9,
- Figure 12 is a front view of the converter of the drug delivery device of Figure 9,
- Figures 13A to 13F illustrate successive steps of operation of the converter of the drug delivery device of Figure 9.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

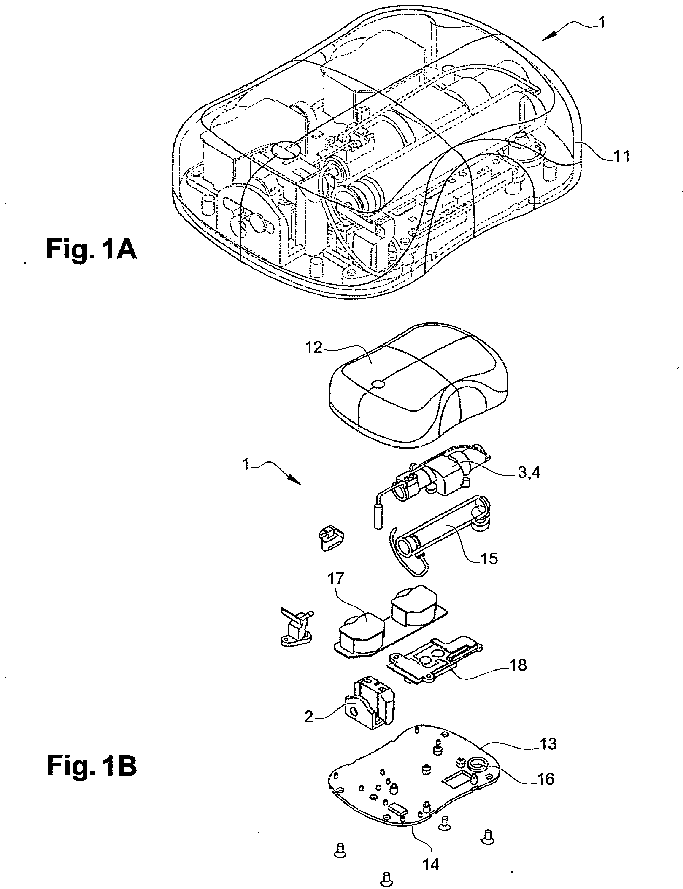

With reference to Figures 1A and 1B is shown a drug delivery device 1 according to an embodiment of the invention. The drug delivery device 1 is of the type having an injection mechanism 2 that is in fluid communication with a reservoir 15 and a pump to deliver medicament to a patient.

Specifically, as shown in Figure 1A, the drug delivery device 1 may be a wearable injector although embodiments are not limited thereto. The drug delivery device 1 may thus be configured to be attached to a patient, for instance to the patient's skin, in order to inject, at a predetermined time, a predetermined volume of a medical product contained within a reservoir 15.

With reference to Figure 1B, the drug delivery device 1 may include a housing 11 formed by a shell 12 and a base 13, and a patch 14 for attaching the base to the patient's skin. The drug delivery device 1 may include various components such as a reservoir 15, for instance in the form of a flexible bag, for containing the medical product, a filling port 16 for filling the reservoir 15 prior to use, a power source 17 such as a battery, an electronic system 18 for controlling the drug delivery device 1 and for triggering automatic injection of a predetermined volume of the medical product at a predetermined time and at a predetermined flow rate, and possibly one or more buttons (not shown) such as an activation button for allowing a user, such as a nurse, to manually trigger insertion of a catheter into an injection site.

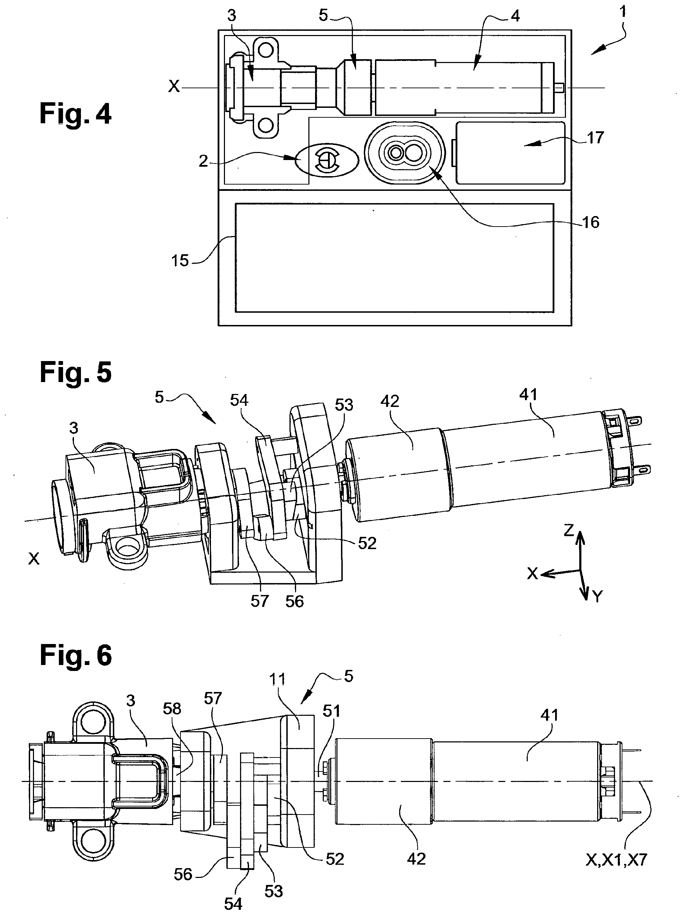

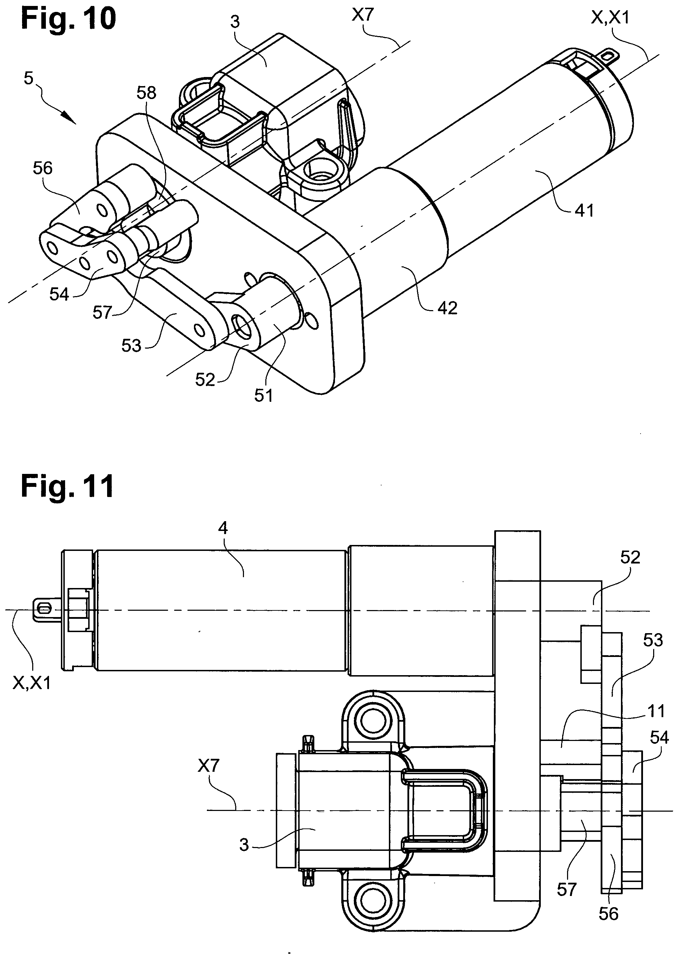

The drug delivery device 1 includes an injection mechanism 2 which may be a catheter insertion mechanism for inserting an inserter needle and a catheter into the injection site, a pump unit 3 including a piston 310 for pumping the medical product out of the reservoir 15, and a motor unit 4for driving the pump unit 3. The motor unit 4 includes a motor 41 having a motor 41 shaft extending along an axis X, and may include a gearbox 42, Figure 5. With reference to Figures 4-5 or 9-10, the drug delivery device 1 includes a converter 5 for converting a continuous one way rotation of the motor 41 shaft into an alternated two-way rotation of the pump piston 310.

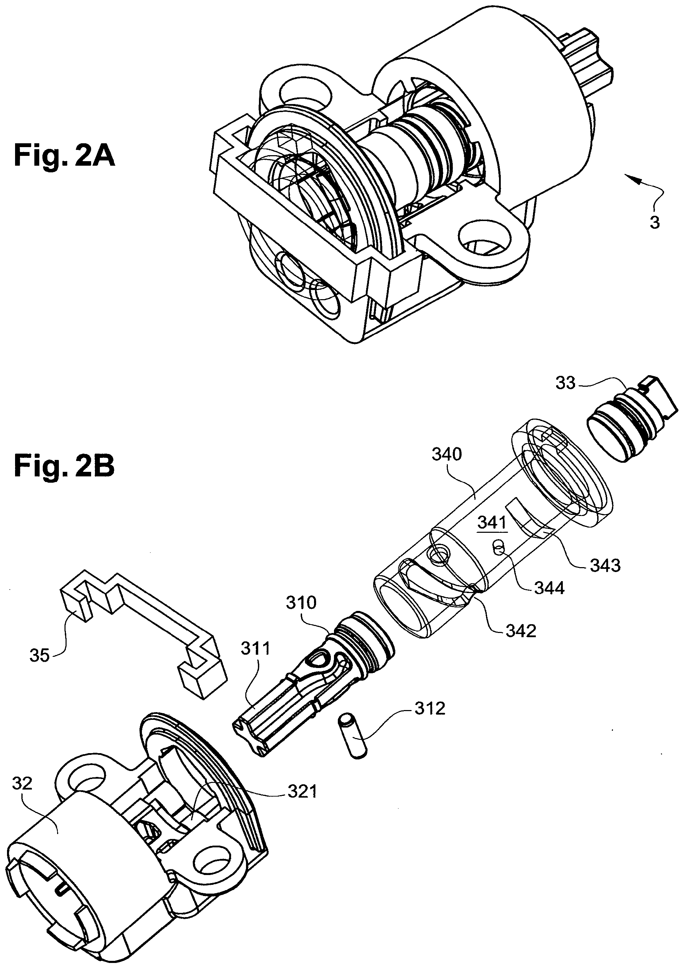

With reference to Figures 2A-2B, the pump unit 3 may be a semi rotary back-and-forth pump and may include some or all of a pump housing 32, a pump bottom 33, a piston 310, and a sleeve 340 delimiting a pump chamber 341 together with the pump button and the piston 310, a piston fin 311 secured to the piston 310 and configured for transmitting torque of a coupling shaft 58 to the piston fin 311, a coupling pin 312 fixed to the piston fin 311 and engaged in a helical groove 342 such that rotation of the piston fin 311 entails a corresponding axial movement of the piston 310 towards the pump bottom 33 (discharge phase) or away from the pump bottom 33 (intake phase), an anti-premature rotation feature 321 arranged on the pump housing 32 to engage a corresponding anti-premature rotation feature 343 of the sleeve 340, a retainer 35 for axially retaining the sleeve 340, and a port 344 for allowing the medical product to flow inside or outside the pump chamber 341. As an example, the pump unit 3 may be similar to the pump disclosed in the patent document US10132308B2.

It is noted that the piston fin 311 is configured to be coupled to the coupling shaft 58 such that rotation of the coupling shaft 58 in one way causes rotation of the piston 310 in the same way, and such that the piston 310 is allowed to slide with respect to the coupling shaft 58 while rotating together with this coupling shaft 58. To that end the piston fin 311 may be X-shaped and may engage a complementarily shaped feature (not shown) of the coupling shaft 58. Therefore, torque of the coupling shaft 58 is transmitted to the piston 310 while the piston 310 is free to axially move with respect to the coulping shaft. The coupling shaft 58 and the piston 310 may be coaxial. The piston fin 311, the coupling and the helical groove 342 form a converter configured for transforming the reciprocated rotation of the coupling shaft 58 into a back-and-forth axial movement of the piston 310.

The anti-premature rotation features 321, 343 are configured to delay rotation of the sleeve 340 so that the sleeve 340 rotates only when the coupling pin 312 abuts against one end of the helical groove 342. Rotation of the sleeve 340 corresponds to a valve state change, i.e. to a positioning of the port 344 in fluid communication with either the reservoir 15 (before a pump intake) or with the injection mechanism 2 (before a pump discharge). The intake step and the discharge step of the pump occur when the coupling pin 312 slides within the helical groove 342, thereby causing axial movement of the piston 310 in one direction or the other (while the sleeve 340 does not rotate due to the anti-premature rotation features 321, 343). The anti-premature features 321, 343 may be, for instance, bumps. The bump 321 may be made of silicon or thermoplastic elastomer, so as to deform under a certain torque. As long as the torque is lower than this certain torque, the bump 323 cannot pass beyond the bump 321. When the torque is higher, the bump 321 deforms and the sleeve 340 can rotate.

In operation, the pump cycle may includes four steps. First, a pump discharge, in which the coupling shaft 58 and the piston 310 rotate in a first rotational direction by a predetermined angle α1 of, for instance, approximately [190°-200°]; a valve state change, in which the coupling shaft 58 and the piston 310 go on rotating in the first rotational direction by a predetermined angle β1 of, for instance, approximately [5°-30°]; a pump intake, in which the coupling shaft 58 and the piston 310 rotate in an opposite second rotational direction by a predetermined angle α2 of, for instance, approximately [190°-200°]; and a valve state change, in which the coupling shaft 58 and the piston 310 go on rotating in the second rotational direction by a predetermined angle β2 of, for instance, approximately [5°-30°]. A total pump cycle may thus require, for instance, 207° of rotation of the piston 310/coupling shaft 58 in each direction, although embodiments are not limited thereto.

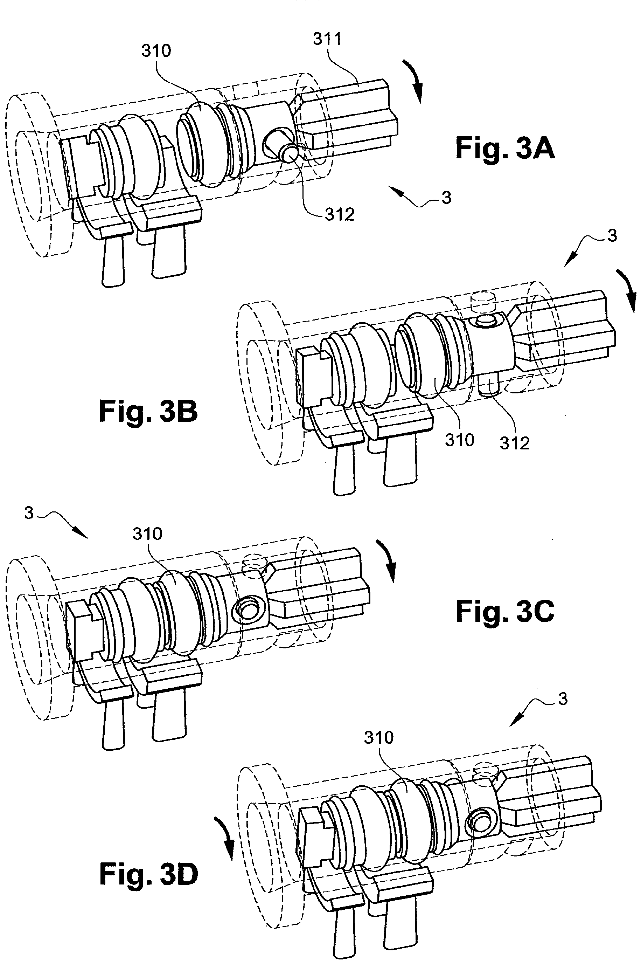

The discharge phase of the pump is illustrated in Figures 3A to 3D. In Figures 3A and 3B, the piston 310 rotates (in the first rotational direction) and slides within the sleeve 340 due to the coupling pin 312 which slides in the helical groove 342. This reduces the volume of the pump chamber 341 so that the medical product exits the pump chamber 341 via the port 344 which is here in fluid communication with the injection mechanism 2. Therefore, the medical product is expelled towards the injection site. It is noted that the sleeve 340 is prevented from prematurely rotating by means of the anti-premature rotation features 321, 343, until the piston 310 is in its top dead center position, Figure 3C, such that all the medical product has been expelled. Once the coupling pin 312 rotates to the end of the helical groove 342, further rotation of the coupling shaft 58/piston 310 causes the coupling pin 312 to overcome the anti-premature rotation features 321, 343, such that the sleeve 340 and the piston 310 rotate together without relative axial translation. The discharge step is over and the valve state change takes place, Figure 3D : the port 344 rotates together with the sleeve 340 to stop fluid communication with the injection mechanism 2 and start fluid communication with the reservoir 15. When the rotational direction of the coupling shaft 58 changes, the intake step begins. The intake phase is quite similar to the discharge step except that the coupling shaft 58 and the piston 310 rotate in the opposite second direction and that the piston 310 slides backwards within the sleeve 340, thereby increasing the volume of the pump chamber 341. Thus, the medical product contained within the reservoir 15 is sucked up into the pump chamber 341. Once the coupling pin 312 rotates to the other end of the helical groove 342, further rotation of the coupling shaft 58/piston 310 causes the coupling pin 312 to overcome the anti-premature rotation features 321, 343, such that the sleeve 340 and the piston 310 rotate together in the second rotational direction without relative axial translation. The intake step is over and another valve state change takes place : the port 344 rotates together with the sleeve 340 to stop fluid communication with the reservoir 15 and start fluid communication with the injection mechanism 2. When the rotational direction of the coupling shaft 58 changes, the discharge step begins.

With reference to Figures 5-6, the movement converter 5 may include a driving shaft 51 connected to the pump unit 3; an upstream crank 52; one or more intermediate connecting rods, such as a first connecting rod 53, a second connecting rod 54, and a third connecting rod 56; a downstream crank 57; and a coupling shaft 58 connected to the piston 310 of the pump unit 3. The movement converter 5 is configured to transform a one way rotation of 360° of the driving shaft 51 into a two-way rotation of a predetermined angle γ of the coupling shaft 58. The predetermined angle γ corresponds to the rotation of the piston 310 in one or the other direction to complete a discharge (or intake) step plus a valve state change. This angle γ may be different than 180°, and may be comprised between ]180°; 360°[(i.e. 180° and 360° excluded). As above-mentioned, the angle γ may be approximately 207°.

The driving shaft 51 extends along an axis X1 which may be parallel to the axis X. Therefore the driving shaft 51 and the motor 41 shaft may be parallel, and possibly coaxial. The driving shaft 51 may be directly or indirectly coupled to the motor 41 shaft. In the illustrated embodiment, the driving shaft 51 is coupled to the motor 41 shaft via a gearbox 42 of the motor unit. In other embodiments, the driving shaft 51 may be directly coupled to the motor 41 shaft, or may be the motor 41 shaft. Anyway, the driving shaft 51 and the motor 41 shaft are configured to rotate at the same time in the same rotational direction. In operation, the driving shaft 51 and the motor 41 shaft perform a continuous single-way rotation.

The coupling shaft 58 extends along an axis X7 which may be parallel to the axis X1. Therefore, the driving shaft 51 and the coupling shaft 58 may be parallel to each other. The coupling shaft 58 is coupled to the piston 310 such that the coupling shaft 58 and the piston 310 are also configured to rotate at the same time in the same rotational direction, and such that the piston 310 can also slide backwards and forwards while rotating together with the coupling shaft 58. In operation, the coupling shaft 58 and the piston 310 perform an alternated two-way rotation.

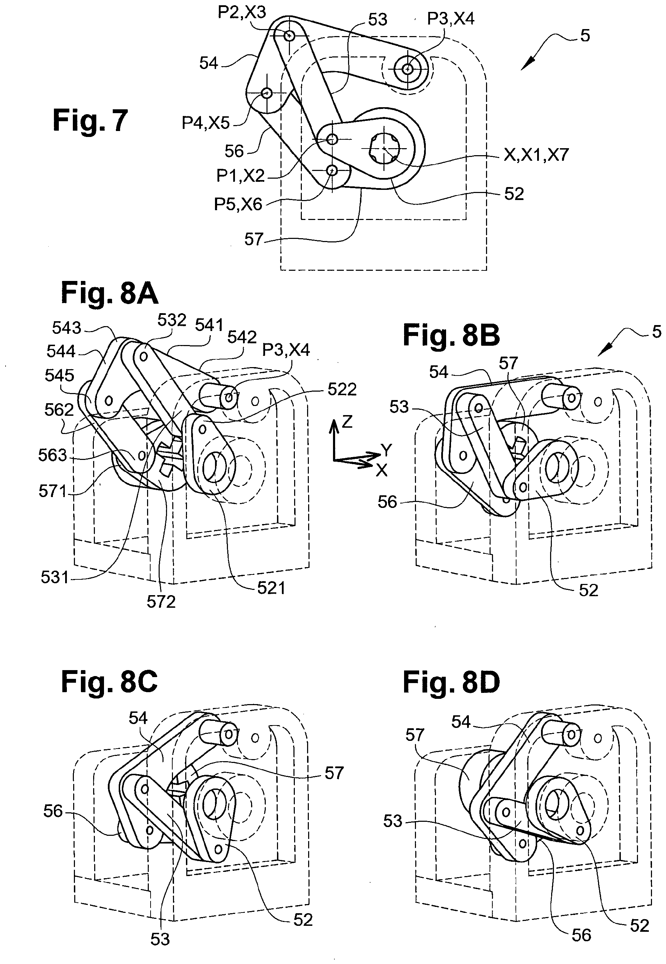

Figures 4 to 8H illustrate the movement converter 5 according to one embodiment of the drug delivery device 1 of the invention. In this embodiment, the driving shaft 51 and the coupling shaft 58 are coaxial. They both extend along the axis X defined by the of the motor 41 shaft. The motor unit 4 may thus be arranged at one side of the movement converter 5, while the pump unit 3 may be arranged at the other side of the movement converter 5.

The upstream crank 52 may be in the form of an elongated rod straightly extending between a first end and 521 an opposite second end 522. The upstream crank 52 extends in the YZ plane, i.e. orthogonal to the axis X, between the driving shaft 51 and the first connecting rod 53. The upstream crank 52 is proximally located with respect to the first connecting rod 53 and connects the driving shaft 51 to the first connecting rod 53. That is, the upstream crank 52 may be interposed between the driving shaft 51 and the first connecting rod 53. The upstream crank 52 is securely connected to the driving shaft 51, for instance at its first end 521, and rotatably connected, for instance at its second end 522, to the first connecting rod 53 by means of a first pivot connection P1. Thus, the upstream crank 52 is rotationnally fixed to the driving shaft 51, such that the upstream crank 52 rotates together with the driving shaft 51, but the upstream crank 52 and the first connecting rod 53 rotate relative to each other around an axis X2 parallel to the axis X1. The axis X2 is offset with respect to the axis X1; they do not coincide with each other. It is contemplated that the downstream crank 57 is the only one which has a full rotation movement, i.e. which rotates 360°, since the upstream crank 52 is the only one which is secured to the driving shaft 51 which continuously rotate in the same single direction around the longitudinal axis X1.

The first connecting rod 53 may be in the form of an elongated rod straightly extending between a first end 531 and an opposite second end 532. The first connecting rod 53 extends in the YZ plane, i.e. orthogonal to the axis X, between the upstream crank 52 and the second connecting rod 54. The first connecting rod 53 is located proximal with respect to the second connecting rod 54 and distal with respect to the upstream crank 52. The first connecting rod 53 connects and may be axially interposed between the upstream crank 52 and the second connecting rod 54. The first connecting rod 53 is rotatably connected to the upstream crank 52, for instance at its first end 531, by means of the first pivot connection P1 and rotatably connected, for instance at its second end 532, to the second connecting rod 54 by means of a second pivot connection P2. Thus, the first connecting rod 53 rotates with respect to the upstream crank 52 around the axis X2, while the first connecting rod 53 and the second connecting rod 54 rotate relative to each other around an axis X3 parallel to the axis X1. The axis X3 is offset with respect to the axis X1 and X2; they do not coincide with one another. The first connecting rod 53 does not excute a full rotation with respect to the second connecting rod 54.

The second connecting rod 54 may be in the form of a bent rod having a first portion 541, which may straithly extend between a first end 542 and an intermediate inflexion point 543, and a second portion 544, which may straithly extend from the intermediate inflexion point to a second end 545. The intermediate inflexion point 543 is arranged between the first end 542 and the second end 545. The second connecting rod 54 may be L-shaped. The second connecting rod 54 may extend in the YZ plane, i.e. orthogonal to the axis X, between the first connecting rod 53 and the third connecting rod 56. The second connecting rod 54 is located proximal with respect to the third connecting rod 56 and distal with respect to the first connecting rod 53. That is, the second connecting rod 54 connects and may be axially interposed between the first connecting rod 53 and the third connecting rod 56. The second connecting rod 54 is rotatably connected to a housing 11 of the motor 41 or of the device 1 by a third pivot connection P3, for instance at its first end 542, rotatably connected to the first connecting rod 53 by the second pivot connection P2, for instance at its intermediate inflexion point 543, and rotatably connected, for instance at its second end 545, to the third connecting rod 56 by means of a fourth pivot connection P4. Therefore, the second connecting rod 54 rotates with respect to the motor 41 around an axis X4 parallel to the axis X1, and rotates with respect to the first connecting rod 53 around the axis X3, and the second connecting rod 54 and the third connecting rod 56 further rotate relative to each other around an axis X5 parallel to the axis X1. The axis X4, X5 are offset with respect to each other, and may be offset with respect to the axis X1, X2, X3; they do not coincide with one another. The second connecting rod 54 does not excute a full rotation with respect to the housing 11, nor with respect to the first connecting rod 53, nor with respect to the third connecting rod 56.

The third connecting rod 56 may be in the form of an elongated rod straightly extending between a first end 561 and an opposite second end 562. The third connecting rod 56 extends in the YZ plane, i.e. orthogonal to the axis X, between the second connecting rod 54 and the downstream crank 57. The third connecting rod 56 is located proximal with respect to the downstream crank 57 and distal with respect to the second connecting rod 54. The third connecting rod 56 connects and may be interposed between the second connecting rod 54 and the downstream crank 57. The third connecting rod 56 is rotatably connected to the second connecting rod 54, for instance at its first end 561, by means of the fourth pivot connection P4 and rotatably connected, for instance at its second end 562, to the downstream crank 57 by means of a fifth pivot connection P5. Thus, the third connecting rod 56 rotates with respect to the second connecting rod 54 around the axis X5, while the third connecting rod 56 and the downstream crank 57 rotate relative to each other around an axis X6 parallel to the axis X1. The axis X6 may be offset with respect to the axis X1 to X5; they do not coincide with one another. The third connecting rod 56 does not excute a full rotation with respect to the second connecting rod 54, and does not excute a full rotation with respect to the downstream crank 57.

It is contemplated that the first connectig rod 53 extends inside the angle formed by the L-shaped second connecting rod 54, i.e. at a concave side of the second connecting rod 54. It is also contemplated that the third connecting rod 56 may also extend at the concave side of the second connecting rod 54.

The downstream crank 57 may be in the form of an elongated rod straightly extending between a first end 571 and an opposite second end 572. The downstream crank 57 extends in the YZ plane, i.e. orthogonal to the axis X, between the third connecting rod 56 and the coupling shaft 58. The downstream crank 57 is distally located with respect to the third connecting rod 56 and connects the third connecting rod 56 to the coupling shaft 58. That is, the downstream crank 57 connects, and may be interposed between the third connecting rod 56 and the coupling shaft 58. The downstream crank 57 is securely connected to the coupling shaft 58, for instance at its second end 572, and rotatably connected, for instance at its first end 571, to the third connecting rod 56 by means of the fifth pivot connection P5. Thus, the downstream crank 57 is rotationnally fixed to the coupling shaft 58, such that the downstream crank 57 rotates together with the coupling shaft 58, but the downstream crank 57 and the third connecting rod 56 rotate relative to each other around the axis X7 which may coincide with the axis X1. It is contemplated that the downstream crank 57 does not execute a full rotation. Instead,the downstream crank 57 rotates around the axis X1/X7 between two angular positions separated by the predetermined angle γ which may, for instance, be equal to 207°, although embodiments are not limited thereto, and has a back-and-forth rotation between these two positions, i.e. rotates 207° in one way and then 207° in the opposite way, and so on.

Although not illustrated in Figures 4 to 8H, to avoid any interference or contact between the connecting rods and cranks, one or more of the upstream crank 52, the first connecting rod 53, the second connecting rod 54, the third connecting rod 56 and the downstream crank 57 may include one or more recesses.

The movement converter 5 of the drug delivery device 1 of the invention thus allows for converting the continuous one-way rotation of the driving shaft 51 into an alternated two-way rotation of the coupling shaft 58. This alternated rotation of the coupling shaft 58, in one way and then in the other, is converted by the connector in an axial back-and-forth movement of the piston 310, as explained earlier.

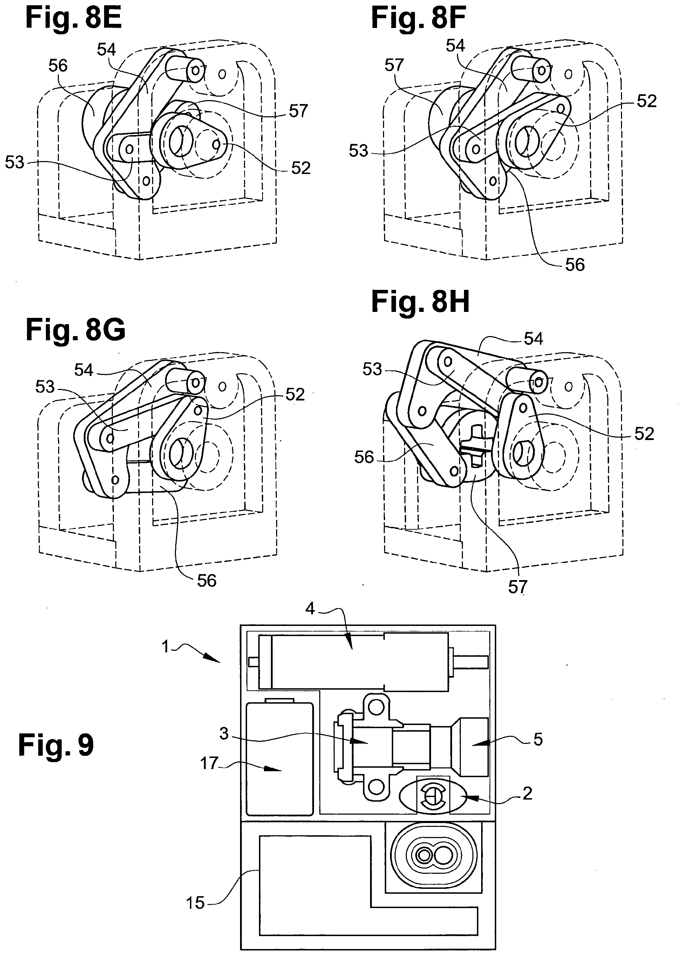

Figures 9 to 13F illustrate the movement converter 5 according to another embodiment of the drug delivery device 1 of the invention. For simplification, the parts or elements of one embodiment which are found identically or similarly in the other embodiment will be identified using the same numerical references and will not be described again. It is reminded that the embodiments are not intended to be mutually exclusive so that the features of one embodiment can be combined with other embodiments as long as they do not contradict each other.

In this embodiment, the driving shaft 51 and the coupling shaft 58 are no longer coaxial, but are rather offset. The driving shaft 51 and the coupling shaft 58 do not coincide with each other, but are arranged parallel to each other in the XY plane. They may even be arranged at 180° relative to each other, such that the converter 5 connects a distal end of the driving shaft 51 to a distal end of the coupling shaft 58. That is, the driving shaft 51 and the coupling shaft 58 may be arranged at a same side of the movement converter 5. Likewise, the motor unit 4and the pump unit 3 may be arranged at a same side of the movement converter 5. The pump unit 3 and the motor unit 4 extend along each other.

The movement converter 5 of Figures 9-13F is similar to the one described in connection with Figures 4-8H, except for the arrangement and/or shape of the uptsream crank, the first connecting rod 53, the second connecting rod 54, the third connecting rod 56 and the downstream crank 57. Indeed, the third connecting rod 56 here is proximally located with respect to the second connecting rod 54, instead of being distally located. Likewise, the downstream crank 57 here is proximally arranged with respect to the third connecting rod 56, instead of being distal to the third connecting rod 56. As a result, the second connecting rod 54 may be the distal-most connecting rod connecting rod of the movement converter 5. The first connecting rod 53 and the second connecting rod 54 may extend in the same YZ plane. The upstream crank 52 and the downstream crank 57 may also extend in the same YZ plane. It is further contemplated that the first connectig rod 53 extends outside the angle formed by the L-shaped second connecting rod 54, i.e. at a convex side of the second connecting rod 54. The third connecting rod 56 may extend at the opposite concave side of the second connecting rod 54.

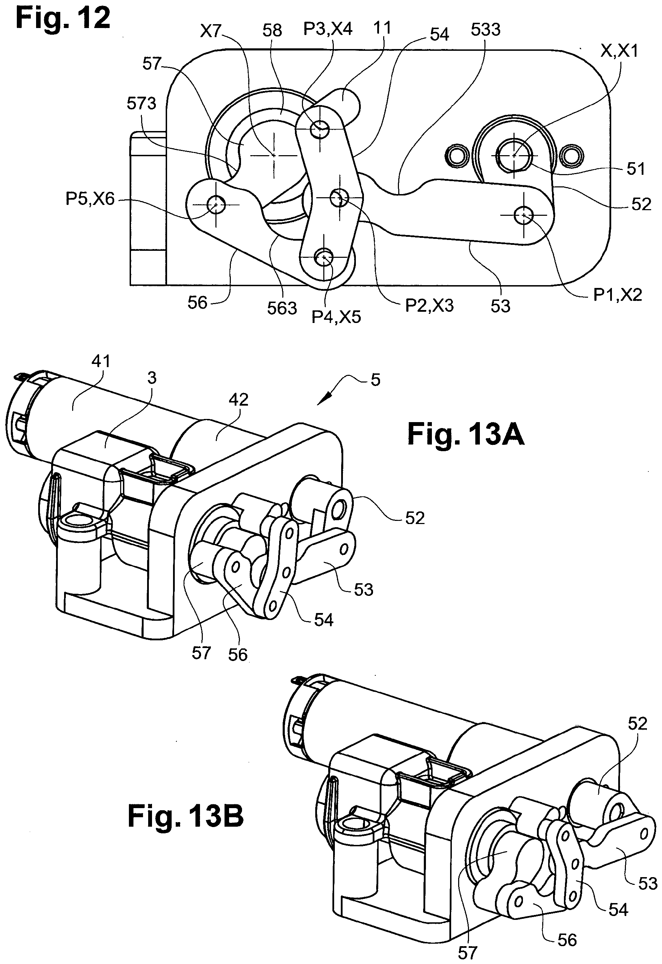

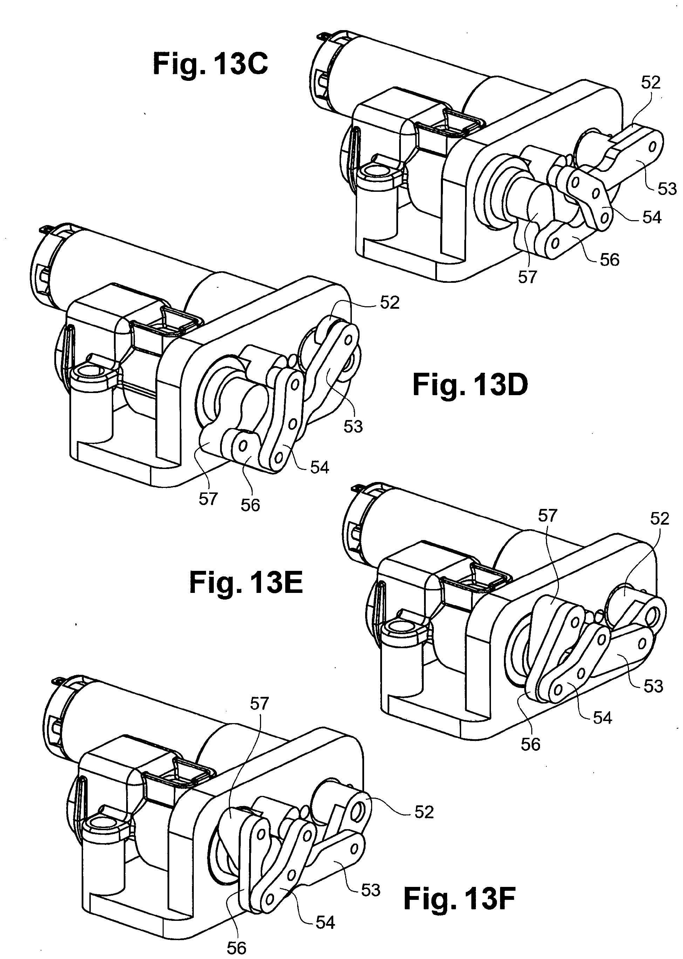

Besides, to avoid any interference or contact between the connecting rods and cranks, one or more of the upstream crank 52, the first connecting rod 53, the second connecting rod 54, the third connecting rod 56 and the downstream crank 57 may include one or more recesses. As illustrated in Figure 12, the first connecting rod 53 may inlcude a lateral recess 533 for avoiding contact with the third connecting rod 56 or the housing 11 to which the third connecting rod 56 is connected by means of the third pivot connection P3. Also, the third connecting rod 56 may include a lateral recess 563 configured for avoiding contact with the first connecting rod 53, and more specifically configured to accommodate the second end of the first connecting rod 53, as shown in Figure 13E. The downstream crank 57 may include a lateral recess 573 too, this lateral recess being configured for avoiding contact between the downstream crank 57 and the portion of the housing 11 which extends towards the intermediate inflexion point of the second connecting rod 54.

The operation of the movement converter 5 according to the embodiment illustrated in Figures 9-13F is otherwise similar to the operation of the movement converter 5 according to the embodiment illustrated in Figures 4-8H. That is, the movement converter 5 of Figures 9-13F also allows for converting the continuous one-way rotation of the driving shaft 51 into an alternated two-way rotation of the coupling shaft 58. This alternated rotation of the coupling shaft 58, in one way and then in the other, causes the axial back-and-forth movement of the piston 310.

It is contemplated that the shape or dimensions of the upstream crank 52, the first connecting rod 53, the second connecting rod 54, the third connecting rod 56 and the downstream crank 57, as well as the location of the pivot connections P1 to P6, and the location of the axis X1 to X7, may vary depending on, for example, the predetermined angle γ that the coupling shaft 58 is required to execute and/or depending on the rotation speed that the coupling shaft 58 is required to have. For instance, the rotation angle of the second connecting rod 54 may be adjusted by modifying one or more of: the relative position of the axis X1 and X4, the distance between the axis X1 and X2, the distance between the axis X2 and X3, the distance between the axis X3 and X4. For instance, the rotation angle of the coupling shaft 58 may be adjusted by modifyng one or more of: the rotation angle of the second crank around the pivot connection P3, the relative position of the axis X4 and X7, the distance between the axis X4 and X5, the distance between the axis X5 and X6, the distance between the axis X6 and X7.

Another aspect of the invention is a method for setting the above-described drug delivery device 1. This method may include the steps of:
determining a targeted flow rate for the medical product at a catheter of the drug delivery device 1,
setting a rotation speed profile for the driving shaft 51.

The rotation speed profile defines the rotation speed of the driving shaft 51 at every time of the pump cycle. This permits to control the rotation speed of the coupling shaft 58. By controlling the rotation speed of the coupling shaft 58, it is possible to define the duration of the pump discharge and the flow rate of the medical product when injected into the injection site. For instance, the rotation speed of the coupling shaft 58 in the one way corresponding to the pump discharge may be decreased so that the duration of the pump discharge increase, thereby reducing the flow rate of the medical product entering into the injection site, thus reducing pain that a patient might feel.

The method also permits to define the duration of the pump intake. This duration may be reduced to be as short as possible, by increasing the rotation speed of the driving shaft 51 (and thus of the coupling shaft 58) when the coupling shaft 58 rotates in the way corresponding to the intake step of the pump.

The method also enables to speed up the valve state changes so that these changes are less time consuming.

It is readily understandable from the above description that the drug delivery device 1 of the invention permits to convert the continuous one-way rotation of the driving shaft 51 into an alternated two-way rotation of the coupling shaft 58, and hence into a back-and-forth movement of the pump piston 310. This allows for avoiding highly accurate and costly control of the driving shaft 51 position at any time, because the driving shaft 51 no longer needs to rotate in one direction and then in the other. Likewise, this allows for reducing the mechanical strains on the motor unit 4and the gearbox 42, thereby improving the lifetime of this motor unit, especially when the drug delivery device 1 is re-usable. It is to be understood that the present invention is not limited to the embodiments described above and illustrated in the drawings; rather, the skilled person will recognize that many changes and modifications may be made within the scope of the appended claims.

## Claims

1. Drug delivery device (1) including:
a housing (11),
a reservoir (15) configured for storing a medical product,
an injection mechanism (2) configured for allowing injection of the medical product into an injection site,
a pump unit (3) for pumping the medical product from the reservoir (15) and expelling this medical product towards the injection site
a motor unit (4) for driving the pump unit (3),
a movement converter (5) including a driving shaft (51) coupled to the motor unit (4) and a coupling shaft (58) coupled to a piston (310) of the pump unit (3), wherein
the movement converter (5) is configured for converting a one-way rotation of the driving shaft (51) into a two-way rotation of the coupling shaft (58).

2. Drug delivery device (1) according to the preceding claim, wherein the movement converter (5) includes an upstream crank (52) fixed to the driving shaft (51), a downstream crank (57) fixed to the coupling shaft (58), and one or more connecting rods rotatably connecting the upstream crank (52) to the downstream crank (57).

3. Drug delivery device (1) according to the preceding claim, wherein the one or more connecting rods include a first connecting rod (53) rotatably mounted on the upstream crank (52) around an axis X2, a second connecting rod (54) rotatably mounted on the first connecting rod (53) an axis X3, and a third connecting rod (56) rotatably mounted on the second connecting rod (54) around an axis X5 and rotatably mounted on the downstream crank (57) around an axis X6.

4. Drug delivery device (1) according to the preceding claim, wherein the second connecting rod (54) is rotatably mounted on the housing (11) around an axis X4.

5. Drug delivery device (1) according to the claim 3 or 4, wherein the second connecting rod (54) includes a first portion (541) extending between the axis X4 and the axis X3, and a second portion (544), inclined with respect to the first portion (541), the second portion (544) extending between the axis X3 and the axis X5.

6. Drug delivery device (1) according to any one of claims 3 to 5, wherein the first connecting rod (53) is disposed between the upstream crank (52) and the second connecting rod (54).

7. Drug delivery device (1) according to any one of claims 3 to 6, wherein the third connecting rod (56) is disposed between the second connecting rod (54) and the downstream crank (57).

8. Drug delivery device (1) according to any one of the preceding claims, wherein the driving shaft (51) and the coupling shaft (58) are parallel.

9. Drug delivery device 1 according to any one of the preceding claims, wherein the driving shaft (51) and the coupling shaft (58) are coaxial.

10. Drug delivery device (1) according to the preceding claim, wherein the second connecting rod (54) is disposed between the first connecting rod (53) and the second connecting rod (54).

11. Drug delivery device (1) according to any one of the preceding claims 1-8, wherein the driving shaft (51) and the coupling shaft (58) are offset.

12. Drug delivery device (1) according to the preceding claim, wherein the first connecting rod (53) and the third connecting rod (56) are disposed at a same side of the second connecting rod (54).

13. Method for setting a drug delivery device (1) according to any one of the preceding claims, the method including the steps of:
determining a targeted flow rate for the medical product at a catheter of the drug delivery device (1),
setting a rotation speed profile for the driving shaft (51).
